# EUROPEAN PATENT APPLICATION

(11) **EP 4 647 748 A1**
(43) Date of publication of application: **12.11.2025**
(21) Application number: 25174581.6
(22) Date of filing: 06.05.2025
(51) Int. Cl.: G01N 27/06, G01N 33/18

(54) **SYSTEMS AND METHODS FOR MONITORING THE CONDUCTIVITY OF A FLUID**

(30) Priority: 06.05.2024 US 202463642995 P
(71) Applicant: Preddio Technologies Inc., North Andover, MA 01845 (US)
(72) Inventor: Ganick, Aaron, Boxford (US)
(74) Representative: Wischmeyer, André

(57) **Abstract**

Systems and methods for monitoring conductivity of a fluid comprising generating a first measurement indicative of the conductivity of the fluid, determining whether a value of the first measurement is within the first calibration range, responsive to determining that the value of the first measurement is not within the first calibration range, calibrating the conductivity sensor with a second calibration range that includes the value of the first measurement, generating a second measurement indicative of the conductivity of the fluid.

## Description

### CROSS-REFERENCE TO REALTED APPLICATIONS

This application claims the benefit of co-pending U.S. Provisional Patent Application No. 63/642,995, filed May 6, 2024, the entire contents of which are incorporated by reference.

### FIELD

This disclosure generally relates to systems and methods for monitoring the conductivity of a fluid. More specifically, this disclosure relates to systems and methods that implement a dynamically calibrated sensing device for monitoring the conductivity of a fluid and/or the total dissolved solid levels of a fluid.

### BACKGROUND

Conductivity sensors are used for monitoring the ionic content, or conductivity, of fluids across various industrial applications to gain insight into fluid composition, concentration, and/or purity. In food and beverage industries, such as the brewing industry, conductivity sensing plays a critical role in maintaining product consistency, enabling automated clean-in-place (CIP) procedures, and ensuring compliance with sanitation standards.

In conventional approaches, conductivity sensors are designed with fixed calibration ranges and/or optimized for sensing a specific fluid or a narrow band of conductivity values. That is, a conductivity sensor is typically calibrated to a static range based on the probe design and/or the application in which the probe is being used. For example, a sensor that is designed to measure medium level conductivities in the general range of 0 to 20,000 µS/cm (the unit of conductivity) may be calibrated against the following three values using a conventional approach: (i) a dry calibration value in which no fluid is present to reduce the impact of electrical noise in the system, (ii) a low calibration value of approximately 1,000 µS/cm, and (iii) a high calibration value of approximately 18,000 µS/cm. In another example, a sensor designed to measure relatively low conductivities (e.g., under 1,000 µS/cm) may be calibrated against the following two values using a conventional approach: (i) a low calibration value of 100 µS/cm and (ii) a high calibration value of 1,000 µS/cm.

However, at least one drawback to calibrating a conductivity sensor to a static range is that a single conductivity sensor is unable to accurately measure the conductivities of fluids with widely varying conductivity levels. This drawback is particularly relevant for instances in which conductivity sensors are deployed in dynamic processing environments, where single pipelines and/or storage tanks are oft used to transport and/or store fluids having dramatically different conductivity profiles.

For example, in the case of a brewery, a single pipeline may be used to sequentially carry beer (which has a moderate conductivity profile), then rinse water (which has a low conductivity profile), then caustic or acidic cleaning solutions (which have conductivity profiles), and then beer again. Because the beer, rinse water, and cleaning solutions have widely varying conductivity levels, a single conductivity sensor deployed in the pipeline and calibrated to a static range cannot be used to accurately monitor the conductivities of each the beer, rinse water, and cleaning solutions. Rather, to effectively monitor the conductivities of beer, rinse water, and cleaning solutions using the conventional approaches described above, a first conductivity sensor calibrated to a fixed, medium conductivity range would need to be deployed in the pipeline for monitoring the conductivity of beer, a second conductivity sensor calibrated to a fixed, low conductivity range would need to be deployed in the pipeline for monitoring the conductivity of rinse water, and one or more third conductivity sensors calibrated to fixed, high conductivity ranges would need to be deployed in the pipeline for monitoring the conductivities of the caustic or acidic cleaning solutions. In that regard, deploying conductivity sensors that are calibrated to static ranges in dynamic processing systems results in increased complexity, increased cost, and limited scalability.

At least another drawback to calibrating a conductivity sensor to a static range is that, as a conductivity sensor becomes less accurate at measuring conductivity levels over time, the points at which the conductivity sensor was calibrated remain the same thereby further decreasing the accuracy of the conductivity sensor.

As the foregoing illustrates, what is needed in the art are more effective techniques for monitoring fluid conductivities.

### SUMMARY

Described herein are techniques for monitoring fluid conductivity with dynamically calibrated sensing devices. For example, with the disclosed techniques, a single conductivity sensing device can be dynamically calibrated as the conductivity sensing device measures the conductivity of a fluid and/or measures the total dissolved solids (TDS) levels of a fluid. Unlike conventional conductivity sensors that rely on static multi-point calibrations, the conductivity sensing device described herein can utilize advanced internal algorithms and/or dynamic calibration routines to measure the conductivity of a fluid and/or the TDS levels of a fluid. For example, the conductivity sensing device can adjust calibration points used for measuring fluid conductivity in real-time based on conductivity values measured by the conductivity sensing device.

When compared to the above-described conventional approaches to monitoring the conductivity of a fluid with a conductivity sensor calibrated to a static range, the conductivity sensing device described herein provides numerous technical advantages. For example, at least one technical advantage of the conductivity sensing device described herein is that the conductivity sensing device generates conductivity measurements with increased accuracy and/or higher resolution. By dynamically calibrating the conductivity sensing device based on the conditions (e.g., conductivity, TDS levels, or some other condition) of the fluid being measured, the conductivity sensing device can provide highly accurate and resolute readings across a broad range of fluid conditions.

At least another technical advantage of the conductivity sensing device described herein is that the conductivity sensing device can be operated with reduced downtime. By connecting the conductivity sensing device to one or more network-connected devices and/or IoT devices, conditions of the fluid being measured can be monitored in real-time thereby reducing the risk of process interruptions and equipment damage. For example, the conductivity sensing device can transmit alerts to one or more network-connected devices and/or IoT devices in real-time as the conductivity and/or TDS levels of the fluid being monitored indicate the occurrence of a process interruption or equipment damage.

At least another technical advantage of using the conductivity sensing device described herein is that product quality can be enhanced when using the conductivity sensing device 104 to monitor the product process. For example, product quality can be more consistently achieved by precisely controlling fluid processing based on the accurate conductivity and/or TDS measurements generated by the conductivity sensing device. In this regard, product quality can more easily comply with health and safety standards.

At least another technical advantage of the using the conductivity sensing device 104 described herein is improved cost efficiency for processes. For example, the accurate conductivity and/or TDS measurements generated by the conductivity sensing device can be used to optimize processes such as, but not limited to, chemical cleaning in place (CIP) and quickly detect problems such as, but not limited, heat exchanger ruptures, thereby significantly reducing operational costs associated with manufacturing processes.

In one independent aspect, a method for monitoring conductivity of a fluid comprising calibrating a conductivity sensor with a first calibration range; generating a first measurement indicative of the conductivity of the fluid; determining whether a value of the first measurement is within the first calibration range; responsive to determining that the value of the first measurement is not within the first calibration range, calibrating the conductivity sensor with a second calibration range that includes the value of the first measurement; and generating a second measurement indicative of the conductivity of the fluid.

In another independent aspect, a conductivity monitoring system comprising a control unit and a conductivity sensing device in electronic communication with the control unit. The conductivity sensing device includes a conductivity sensor and a processor adapted to receive calibration data from the control unit; calibrate, based on the calibration data, the conductivity sensor with a first calibration range; receive a first measurement indicative of the conductivity of a fluid from the conductivity sensor; determine whether a value of the first measurement is within the first calibration range; and responsive to determining that the value of the first measurement is not within the first calibration range, calibrating a conductivity sensor with a second calibration range that includes the value of the first measurement.

In another independent aspect, a conductivity sensing device comprising a conductivity sensor adapted to sense a conductivity value of a fluid; a memory device adapted to store calibration data; and a processor in electronic communication with the conductivity sensor and the memory device. The processor is adapted to calibrate, based on the calibration data, the conductivity sensor with a first calibration range; receive the conductivity value of the fluid from the conductivity sensor; determine whether the conductivity value of the fluid is within the first calibration range; and responsive to determining that the conductivity value of the fluid is not within the first calibration range, calibrate a conductivity sensor with a second calibration range that includes the conductivity value of the fluid.

Other aspects will become apparent by consideration of the detailed description and accompanying drawings.

### BRIEF DESCRIPTION OF THE DRAWINGS

FIG. 1 is a block diagram of a system for monitoring the conductivity of a fluid, according to various embodiments.
FIG. 2 is a block diagram of an example conductivity sensing device 104, according to various embodiments.
FIG. 3 illustrates an example conductivity sensing device, according to various embodiments.
FIG. 4 is a flow diagram of method steps for monitoring the conductivity of fluid, according to various embodiments.
FIGS. 5A-5C illustrate an example process in which a conductivity sensor monitors multiple fluids flowing through a single pipeline, according to various embodiments
FIG. 6 illustrates an example in which a conductivity sensor detects a leak from a heat exchanger, according to various embodiments.
FIG. 7 is a flow diagram of method steps for monitoring the conductivity of fluid during a fluid manufacturing process, according to various embodiments.
FIG. 8 is a flow diagram of method steps for calibrating a conductivity sensor, according to various embodiments.

### DETAILED DESCRIPTION

Before any embodiments are explained in detail, it is to be understood that the embodiments are not limited in its application to the details of the configuration and arrangement of components set forth in the following description or illustrated in the accompanying drawings. The embodiments are capable of being practiced or of being carried out in various ways. Also, it is to be understood that the phraseology and terminology used herein are for the purpose of description and should not be regarded as limiting. The use of "including," "comprising," or "having" and variations thereof are meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings.

In addition, it should be understood that embodiments may include hardware, software, and electronic components or modules that, for purposes of discussion, may be illustrated and described as if the majority of the components were implemented solely in hardware. However, one of ordinary skill in the art, and based on a reading of this detailed description, would recognize that, in at least one embodiment, the electronic-based aspects may be implemented in software (e.g., stored on non-transitory computer-readable medium) executable by one or more electronic processors, such as a microprocessor and/or application specific integrated circuits ("ASICs"). As such, it should be noted that a plurality of hardware and software-based devices, as well as a plurality of different structural components, may be utilized to implement the embodiments. For example, "servers," "computing devices," "controllers," "processors," etc., described in the specification can include one or more electronic processors, one or more computer-readable medium modules, one or more input/output interfaces, and various connections (e.g., a system bus) connecting the components.

Relative terminology, such as, for example, "about," "approximately," "substantially," etc., used in connection with a quantity or condition would be understood by those of ordinary skill to be inclusive of the stated value and has the meaning dictated by the context (e.g., the term includes at least the degree of error associated with the measurement accuracy, tolerances [e.g., manufacturing, assembly, use, etc.] associated with the particular value, etc.). Such terminology should also be considered as disclosing the range defined by the absolute values of the two endpoints. For example, the expression "from about 2 to about 4" also discloses the range "from 2 to 4." The relative terminology may refer to plus or minus a percentage (e.g., 1%, 5%, 10%, or more) of an indicated value.

Functionality described herein as being performed by one component may be performed by multiple components in a distributed manner. Likewise, functionality performed by multiple components may be consolidated and performed by a single component. Similarly, a component described as performing particular functionality may also perform additional functionality not described herein. For example, a device or structure that is "configured" in a certain way is configured in at least that way but may also be configured in ways that are not explicitly listed.

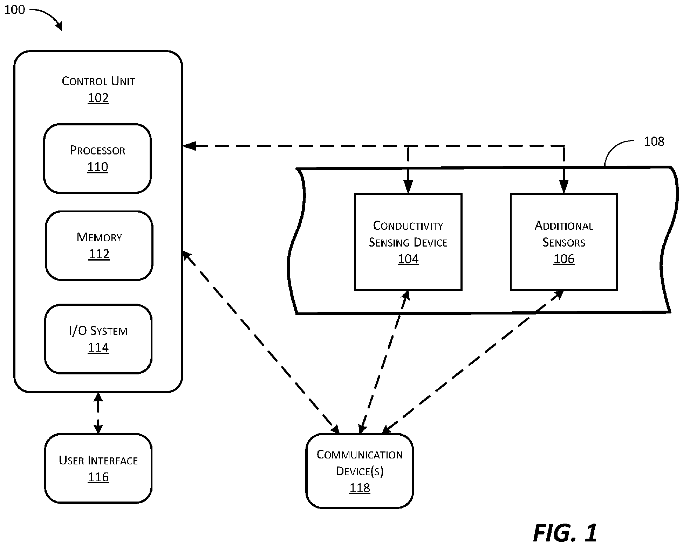

FIG. 1 is a block diagram of a system 100 for monitoring the conductivity of a fluid, according to various embodiments. As will be described in more detail herein, the system 100 can be implemented to monitor the conductivity and/or TDS levels of fluids across a variety of industries such as, but not limited to, the food and beverage manufacturing industry. Moreover, within the food and beverage manufacturing industry, the system 100 described herein can be implemented across a variety of applications. For example, without limitation, the system 100 can be used to measure the conductivity and/or TDS levels of boiler feed water to verify whether the water is of the correct quality, thereby preventing scale buildup and improving efficiency of the boiler. As another non-limiting example, the system 100 can be used to monitor the conductivity and/or TDS levels of the rinse water used in cleaning processes to verify whether the water is clean, thereby ensuring compliance with health and safety standards. As another non-limiting example, the system 100 can be used to monitor the purity of the water used in manufacturing processes, thereby helping maintain product quality and safety. As another non-limiting example, the system 100 can be used to monitor the conductivity and/or TDS levels of cleaning chemicals to assess the concentration and/or effectiveness of the cleaning chemicals, thereby optimizing cleaning processes and ensuring thorough sanitation. As another non-limiting example, the system 100 can be used to monitor the conductivity and/or TDS levels of fluids to detect ruptures and/or leaks in heat exchangers, thereby preventing cross-contamination and facilitating timely repairs. As another non-limiting example, the system 100 can be used to monitor the concentration of solids in fluids used in various processes associated with the manufacture of food and beverages

As shown in FIG. 1, the system 100 includes a central computing device, or control unit, 102 that is communicatively coupled to a conductivity sensing device 104 and one or more additional sensors 106. The conductivity sensing device 104 is adapted to monitor the conductivity and/or TDS levels of one or more fluids being processed in a process controlled by the control unit 102. As used herein, the term "monitoring" can refer to the conductivity sensing device 104 generating measurements and/or sensing values indicative of the conductivity and/or TDS levels of a fluid. The one or more additional sensors 106 can include, for example, one or more temperatures sensors, one or more vibration sensors, one or more pressure sensors, and/or one or more other types of sensors. Although shown as separate devices in the illustrated example of FIG. 1, in some examples, the one or more additional sensors 106 are integrated within the conductivity sensing device 104.

In the illustrated example of FIG. 1, the conductivity sensing device 104 and the one or more additional sensors 106 are shown to be installed within a pipe 108. In that regard, the conductivity sensing device 104 monitors the conductivity and/or TDS levels of fluids flowing through the pipe 108. However, persons skilled in the art should understand that the conductivity sensing device 104 and/or the one or more additional sensors 106 can additionally and/or alternatively be installed in one or more other types of devices adapted for transporting and/or storing fluids (e.g., tanks, reservoirs, cisterns, etc.).

The control unit 102, which can be implemented as any suitable computing device (e.g., desktop computer, laptop, server, smartphone, tablet, etc.) and/or cloud-based computing device, includes a processor 110, a memory 112, an input/output ("I/O") system 114, and a user interface 116 that are interconnected by a bus. The I/O system 114 includes routines for transferring information between components within the control unit 102 and other components of the system 100. For example, the I/O system 114 includes a communication interface that is configured to provide communication between the control unit 102 and the conductivity sensing device 104. As another example, the communication interface of the I/O system 114 provides communication between the control unit 102 and the one or more additional sensors 106. In some examples, the communication interface of the I/O system 114 communicates with the conductivity sensor 104 and/or the one or more additional sensors 106 via one or more intermediary communication devices 118. The one or more intermediary communication devices 118 can include, for example, one or more network hubs, repeaters, bridges, switches, routers, gateways, and/or other network-connected computing devices.

The communication interface of the I/O system 114 enables the control unit 102 to communicate with the conductivity sensing device 104, the one or more additional sensors 106, and/or the one or more intermediary communication devices 118 through a wireless connection. The wireless connection can be enabled via a network, for example, a wide area network (WAN) (e.g., the Internet, a TCP/IP based network, a cellular network, such as, for example, a Global System for Mobile Communications [GSM] network, a General Packet Radio Services [GPRS] network, a Code Division Multiple Access [CDMA] network, an Evolution-Data Optimized [EV-DO] network, an Enhanced Data Rates for GSM Evolution [EDGE] network, a 3 GSM network, a 4GSM network, a Digital Enhanced Cordless Telecommunications [DECT] network, a Digital AMPS [IS-136/TDMA] network, or an Integrated Digital Enhanced Network [iDEN] network, etc.). In other examples, the network is, for example, a local area network (LAN), a neighborhood area network (NAN), a home area network (HAN), or personal area network (PAN) employing any of a variety of communications protocols, such as Wi-Fi, Bluetooth, ZigBee, etc. In some examples, the network includes one or more of a wide area network (WAN), a local area network (LAN), a neighborhood area network (NAN), a home area network (HAN), or personal area network (PAN).

The memory 112 includes, for example, a read-only memory ("ROM"), a random access memory ("RAM"), an electrically erasable programmable read-only memory ("EEPROM"), a flash memory, a hard disk, an SD card, or another suitable magnetic, optical, physical, or electronic memory device. The memory 112 stores software, such as but not limited to firmware, one or more applications, program data, one or more program modules, and/or other executable instructions, for calibrating a conductivity sensing device 104. For example, the memory 112 can store software that includes one or more algorithms, methods, and/or instructions for adjusting, based on measurements taken by the conductivity sensing device 104, based on measurements taken by one or more additional sensors 106, and/or based on the current context of the process in which conductivity sensing device 104 is monitoring, one or more calibration points and/or a calibration range of the conductivity sensing device 104. In some examples, the memory 112 further stores software for controlling a process being monitored by the conductivity sensing device 104.

In some examples, the memory 112 further stores calibration data that can be used for adjusting one or more calibration setpoints of the conductivity sensing device 104. For example, the memory 112 can store calibration points and/or ranges for the conductivity sensing device 104 in the form of a table and/or some other suitable format. As used herein, the phrase "calibration points" refers to the conductivity points, or values, to which the conductivity sensing device 104 is calibrated to measure. Likewise, the phrase "calibration range" refers to the range of calibration values that the conductivity sensing device 104 is calibrated to measure.

In some examples, the calibration data includes respective calibration points and/or ranges for a conductivity sensing device 104 that are specific to fluids being monitored. For example, the calibration data includes a first set of calibration points and/or ranges that are used by the conductivity sensing device 104 to monitor the conductivity of a first fluid (e.g., beer), a second set of calibration points and/or ranges that are used by the conductivity sensing device 104 to monitor the conductivity of a second fluid (e.g., rinse water), a third set of calibration points and/or ranges that are used by the conductivity sensing device 104 to monitor the conductivity of a third fluid (e.g., cleaning solution), and so on. In some examples, the calibration data includes respective calibration points and/or ranges for a conductivity sensing device 104 that are associated with a particular process and/or step in a process involving a fluid. In some examples, the calibration data can additionally and/or alternatively be stored on the conductivity sensing device 104. As will be described in more detail herein, the control unit 102 can adjust one or more calibration points of the conductivity sensing device 104 using the calibration data stored in memory 112.

In some examples, the conductivity sensing device 104 includes and/or is coupled to a memory device that stores calibration data that can be used for adjusting one or more calibration setpoints of the conductivity sensing device 104. In such examples, the conductivity sensing device 104 can adjust one or more of its own calibration points 104 using the calibration data stored in the memory device.

As will be described in more detail herein, during operation of the system 100, the conductivity sensing device 104 can be dynamically calibrated while monitoring the conductivity and/or TDS levels of a fluid. Unlike conventional conductivity probes that rely on static multi-point calibrations, the conductivity sensing device 104 described herein can utilize algorithms and/or dynamic calibration routines to recalibrate while measuring the conductivity of a fluid and/or the TDS levels of a fluid. For example, the conductivity sensing device 104 can adjust calibration points used for measuring fluid conductivity in real-time based on conductivity values measured by the conductivity sensing device 104. In some examples, the conductivity sensing device 104 can adjust calibration points based on one or more instructions received from the control unit 102.

In operation, a processor included in and/or coupled to the conductivity sensing device 104 can adjust a current calibration of the conductivity sensing device 104 using the calibration data. For example, when the conductivity sensing device 104 generates a conductivity measurement for a fluid sample, the processor can compare the conductivity measurement against calibration points included in the one or more calibration points and/or ranges stored in memory. The processor then selects, based on this comparison, calibration points from the calibration data stored in memory and adjusts the calibration of the conductivity sensing device 104 with the calibration points. Then, with the newly selected calibration points, the conductivity sensing device 104 can generate a new, more accurate and/or resolute conductivity measurement of the fluid sample. In other words, the conductivity sensing device 104 can generate a conductivity measurement with higher accuracy and resolution once properly calibrated.

In some examples, the conductivity sensing device 104 is connected to can be connected to a network and/or the Internet of Things (IoT). In such examples, the conductivity sensing device 104 can additionally adjust calibration points based on information received from one or more other network-connected devices and/or IoT devices. Furthermore, in such examples, the conductivity sensing device 104 can transmit conductivity and/or TDS level measurements to one or more other network-connected devices and/or IoT devices while the conductivity sensing device 104 is generating the measurements and/or after the conductivity sensing device 104 generates the measurements. In some examples, the network-connected devices and/or IoT devices include the control unit 102, one or more other conductivity sensing devices 104, and/or one or more additional sensors 106.

### DYNAMIC CALIBRATION EXAMPLE

In a non-limiting example, the conductivity sensing device 104 operates in accordance with a first calibration range of 1,000 µS/cm to 18,000 µS/cm. While operating in accordance with this first calibration range, the conductivity sensing device 104 generates a conductivity measurement for a fluid sample that is equal to 800 µS/cm. The processor included in and/or coupled to the conductivity sensing device 104 then determines whether the conductivity measurement of 800 µS/cm is outside of the first calibration range and/or skews towards the lower end of the first calibration range. In response to determining that the conductivity measurement of 800 µS/cm is outside of the first calibration range, with the disclosed techniques, the processor adjusts the calibration range of the conductivity sensing device 104 to more closely align with the conductivity measurement of 800 µS/cm. In that regard, the processor selects one or more new calibration points from the calibration data stored in the memory of the conductivity sensing device 104 and configures the conductivity sensing device 104 with the new calibration points. For example, the processor selects and configures the conductivity sensing device 104 with new calibration points that define a range between 100 µS/cm and 1,000 µS/cm.

Then, while configured with the new calibration points, the conductivity sensing device 104 can generate a new conductivity measurement for the fluid sample. If the value of the new conductivity measurement is within the range defined by the new calibration points, the processor can determine that this new conductivity measurement satisfies an accuracy threshold and stops adjusting the calibration of the conductivity sensing device 104. For example, if the new conductivity measurement has a value of 400 µS/cm, which is between the low point (e.g., 100 µS/cm) and the high point (e.g., 1,000 µS/cm) of the range defined by the new calibration points, the processor will stop recalibrating the conductivity sensing device 104.

However, if the new conductivity measurement is greater than the highest point of the range defined by the new calibration points, the processor can increase calibration points of the conductivity sensing device 104 and/or move the boundaries of the range defined by the calibration points of the conductivity sensing device 104 until new conductivity measurements fall within an updated calibration range. Likewise, if the new conductivity measurement is less than the lowest point of the range defined by the new calibration points, the processor can decrease the calibration points of the conductivity sensing device 104 and/or move the boundaries of the range defined by the calibration points of the conductivity sensing device 104 until new conductivity measurements fall within an updated calibration range.

To avoid entering an infinite loop of adjusting the calibration of the conductivity sensing device 104, in some examples, the processor can select a new calibration range from a list of pre-defined calibration ranges included in the calibration data based on one or more of technical specifications of the probe included in the conductivity sensing device 104, properties of the fluid being measured, a current step in the process being monitored by the conductivity sensing device 104 (e.g., a rinsing step, a cleaning step, etc.), and/or other information associated with the specific application in which conductivity of a fluid is being measured. In some examples, users can define one or more custom calibration ranges that are specific to a particular application. For example, when measuring the conductivity of a fluid that is expected to have a conductivity between 114 µS/cm to 875 µS/cm for a particular application, the user can define a calibration range based on this expectation.

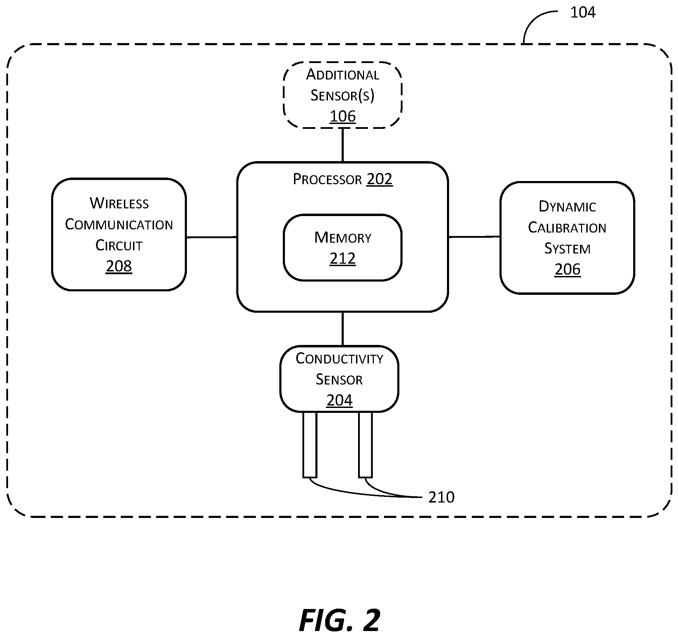

FIG. 2 is a block diagram of an example conductivity sensing device 104, according to various embodiments. As shown, the conductivity sensing device 104 includes a processor 202 that is coupled to a conductivity sensor 204, a dynamic calibration system 206, and a wireless communication circuit 208. In some examples, the conductivity sensing device 104 further includes and/or is coupled to the one or more additional sensors 106 described herein. In such examples, the one or more additional sensors 106 are coupled to the processor 202 and can be integrated within and/or attached to the conductivity sensor 104. The one or more additional sensors 104 can include, for example, a vibration sensor, an accelerometer, a temperature sensor, a pressure sensor, and/or some other type of sensor.

The conductivity sensor 204 includes probes 210 (e.g., electrodes) that can sense and/or generate measurements indicative of the conductivity of a fluid. In some examples, the probes 210 can also sense and/or generate measurements indicative of the TDS levels of a fluid. However, as the TDS levels of a fluid can be correlated with the conductivity of the fluid, in some examples, the TDS levels of the fluid may not be sensed directly and instead can be determined based on the conductivity measurements generated by the one or more probes 210. Although illustrated as including two probes 210 in the example of FIG. 2, in other examples, the conductivity sensor 204 can include more than two probes 210 (e.g., three probes, four probes, etc.). Hereinafter, one or more functions described as being performed by the probes 210 (e.g., sensing conductivity of a fluid, generating conductivity measurements, etc.) can more generally be described as being performed by the conductivity sensor 204. Moreover, any of the functions described herein as being performed by the conductivity sensor 204 (e.g., sensing conductivity of a fluid, generating conductivity measurements, etc.) can more generally be described as functions being performed by the conductivity sensing device 104.

The processor 202 is adapted to receive measurements, such as conductivity and/or TDS level measurements, indicative of one or more conditions of a fluid (e.g., a fluid flowing through pipe 108) from the conductivity sensor 204 and determine, based on the received measurements, whether to adjust one or more calibration points of the conductivity sensing device 104. For example, the processor 202 can determine whether to adjust one or more calibration points of the conductivity sensing device 104 by comparing the measurements indicative of a current condition (e.g., conductivity, TDS levels, or some other condition) of the fluid to expected values of the condition of the fluid. In response to determining to adjust one or more calibration points of the conductivity sensing device 104, the processor 202 can select one or more new calibration points from calibration data stored in memory 212. For example, the processor 202 selects one or more new calibration points based on the value(s) of the measurements indicative of a current condition of the fluid. Moreover, the processor 202 then configures the conductivity sensor 204 with the newly selected calibration points. In that regard, the processor 202 dynamically calibrates the conductivity sensing device 104 based on measurements generated by the conductivity sensor 204. While the conductivity sensing device 104 is configured with the one or more new calibration points, the conductivity sensor 204 can generate new measurements that more accurately reflect a current condition of the fluid.

As a non-limiting example, (i) if the conductivity sensor 204 senses a conductivity value of approximately 500 µS/cm and (ii) 500 µS/cm lies outside of the current calibration range of the conductivity sensing device 104, the processor 202 can select new calibration points that are centered around and/or otherwise includes 500 µS/cm. In some examples, the processor 202 can determine that a conductivity value of approximately 500 µS/cm corresponds to a process change (e.g., from cleaning to rinsing) and selects one or more new calibration points based on the process change.

In some examples, the processor 202 uses the dynamic calibration system 206 to select and configure the conductivity sensing device 104 with one or more new calibration points. In some examples, the dynamic calibration system 206 implements an algorithm and/or a machine learning model for dynamically calibrating the conductivity sensing device 104. In such examples, the dynamic calibration system 206 can update calibration data and/or other information associated with operation of the conductivity sensing device 106 after each measurement cycle of the conductivity sensing device 104. Although shown as being a separate components, in some examples, the dynamic calibration system 206 is included in and/or integrated with the processor 202 and/or memory 212. In some examples, the dynamic calibration system 206 can determine one or more new calibration points for the conductivity sensing device 104 based in part on measurements generated by one or more additional sensors 106.

In some examples, to avoid repeated re-calibration of the conductivity sensing device 104, the processor 202 and/or a remote computing device (e.g., the control unit 102) can perform post processing on measurements generated by the conductivity sensor 104. For example, the processor 202 stops re-calibrating the conductivity sensing device 104 after a predetermined number of re-calibrations (e.g., 3, 5, etc.). In this example, measurements generated by the conductivity sensor 202 can be post-processed against one or more additional calibration sets.

As shown, the processor 202 is coupled to and/or includes the memory 212, which stores instructions and/or programs that can be executed by the processor 202 for dynamically calibrating the conductivity sensing device 104. For example, the memory 212 stores one or more algorithms that can be executed by the processor to analyze the measurements generated by the conductivity sensor 204 and/or to determine whether to adjust a calibration point of the conductivity sensing device 104. Moreover, in some examples, the memory 212 can store calibration data that can be used by the processor to adjust calibration points of the conductivity sensing device 104.

As described herein, calibration data can be used for adjusting one or more calibration setpoints of the conductivity sensing device 104. As described herein, calibration data can include calibration points and/or ranges for the conductivity sensing device 104 organized in the form of a table and/or some other suitable format. In some examples, the calibration data includes respective calibration points and/or ranges for a conductivity sensing device 104 that are specific to fluids being monitored. For example, the calibration data includes a first set of calibration points and/or ranges that are used by the conductivity sensing device 104 to monitor the conductivity of a first fluid (e.g., beer), a second set of calibration points and/or ranges that are used by the conductivity sensing device 104 to monitor the conductivity of a second fluid (e.g., rinse water), a third set of calibration points and/or ranges that are used by the conductivity sensing device 104 to monitor the conductivity of a third fluid (e.g., cleaning solution), and so on. In some examples, the calibration data includes respective calibration points and/or ranges for a conductivity sensing device 104 that are associated with a particular process and/or step in a process involving a fluid. In some examples, the calibration data includes one or more conductivity measurement values that may be indicative of a fault condition (e.g., a leak, a component failure, contamination, etc.). In some examples, the calibration data includes information associated with expected conductivity measurement values for respective steps in one or more processes involving one or more fluids.

In some examples, the memory 212 further stores a dynamic algorithm and/or machine learning model that can be trained and used by the processor 202 to determine new calibration points and/or ranges for the conductivity sensing device 104. In such examples, the dynamic algorithm and/or machine learning model can be updated in real-time based on one or more of the conductivity measurements generated by the conductivity sensor 204, the measurements generated by one or more additional sensors 106, contextual information associated with the process being monitored by the conductivity sensing device 104, and/or one or more other parameters.

Moreover, as described herein, in some examples the memory 212 stores and/or includes the dynamic calibration system 206. The dynamic calibration system automatically adjusts the calibration points of the conductivity sensing device 104 based on one or more of real-time measurements, such as conductivity and/or TDS level measurements, generated by the conductivity sensor 104, measurements generated by one or more additional sensors 106 (e.g., vibration measurements pressure measurements, temperature measurements, etc.) that are indicative of a current step in a process involving the fluid, and/or calibration data. In that regard, the dynamic calibration system 206 helps the conductivity sensing device 104 to generate measurements of both higher accuracy and resolution for a variety of fluid conditions and/or types. In some examples, the dynamic calibration system 206 can be implemented as one or more of a software application, a computer program, or a set of instructions executed by the processor 202.

In some examples, the processor 202 generates one or more alerts in response to determining that a conductivity measurement generated by conductivity sensor 204 is indicative of a fault condition. For example, the processor 202 can determine that a fault condition is present when the value of a conductivity measurement generated by the conductivity sensor 204 differs from an expected conductivity value (e.g., stored in the calibration data) by more than a threshold amount. In response to detecting the presence of a fault condition, the processor 202 can generate an alert. In some examples, generating an alert includes triggering a visual and/or audible indicator. In some examples, generating an alert includes transmitting an alert notification to a network connected device, such as the control unit 102.

In some examples, the processor 202 is further adapted to determine whether changes in conductivity measurements correspond to an expected transition between steps in a process being monitored by the conductivity sensing device 104. For example, the processor 202 can determine whether a change in conductivity value is expected based on (i) calibration data indicative expected conductivities values for fluids during different steps in a process and (ii) real-time measurements generated by the conductivity sensor 204. If the processor 202 determines that a change in conductivity measurements is attributed to an expected transition between steps in a process being monitored by conductivity sensing device 104, the conductivity sensing device 104 continues operating as normal. However, if the processor 202 determines that a change in conductivity measurements is attributed to an expected transition between steps in a process being monitored by conductivity sensing device 104, the processor 202 can generate an alert indicative of a fault condition.

With reference to FIG. 2, the wireless communication circuit 208 allows the conductivity sensing device 104 to send data (e.g., conductivity and/or TDS level measurements) and alerts to various network-connected and/or IoT devices. For example, the wireless communication circuit 208 can transmit data and/or alerts to the control unit 102, thereby facilitating immediate action or adjustments to manufacturing processes.

In some examples, the wireless communication circuit 208 can be used to receive new calibration points, calibration ranges, and/or sets of calibration points from a network-connected device and/or an IoT device. For example, the processor 202 can receive, via the wireless communication circuit 208, one or more new calibration points for the conductivity sensing device 104 from the control unit 102. In some examples, the processor 202 transmits, using the wireless communication circuit 208, a request to the control unit 102 for calibration data. In such examples, the control unit 102 transmits the calibration data to the conductivity sensing device 104 and the processor 202 determines, based on the received calibration data, one or more new calibration points and/or ranges for the conductivity sensing device 104. In some examples, the request for calibration data is implicit. In such examples, the processor 202 transmits one or more conductivity measurements generated by the conductivity sensor 204 to the control unit 102 and receives calibration data from the control unit 102 in response.

In some examples, the control unit 102 can determine the one or more new calibration points and/or ranges for the conductivity sensing device 104. In such examples, the processor 202 transmits, via the wireless communication circuit 208, one or more conductivity measurement values to the control unit 102. Then, based on the received measurement values and the calibration data stored on the control unit 102, the control unit 102 determines one or more new calibration points and/or ranges for the conductivity sensing device 104 and transmits the one or more new calibration points and/or ranges to the conductivity sensing device 104. The processor 202 then receives, via the wireless communication circuit 208, the one or more new calibration points and/or ranges from the control unit 102 and configures the conductivity sensing device 104 with the one or more new calibration points and/or ranges.

In some examples, the control unit 102 can implement the dynamic algorithm and/or machine learning model described herein with respect to the conductivity sensing device 102. In such examples, the control unit 102 periodically and/or intermittently updates the calibration data stored thereon based on values of calibration points and/or ranges that have been implemented by one or more conductivity sensing device 104 connected to the IoT. In some examples, the control unit 102 can update and/or modify calibration data each time one or more calibration points for a particular conductivity sensing device 104 connected to the IoT are adjusted.

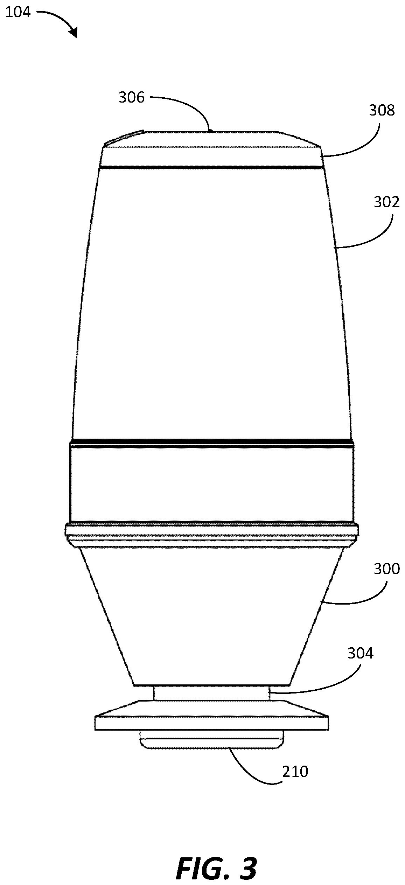

FIG. 3 illustrates an example conductivity sensing device 104, according to various embodiments. In the illustrated example of FIG. 3, the conductivity sensing device 104 includes a metal injection molded stainless steel housing 300. The conductivity sensing device 104 further includes a removable top cover 302, which can be constructed from an ultraviolet rated polycarbonate material. The removable top cover 302 can be removed from the stainless-steel housing 300 to allow a user to change a battery of the conductivity sensing device 104. Together, the stainless steel housing 300 and removable top cover 302 protect one or more of the components included in the conductivity sensing device 104. For example, the stainless steel housing 300 and removable top cover 302 protect one or more of the processor 202, the conductivity sensor 204, the dynamic calibration system 206, the wireless communication circuit 208, the memory 212, and/or one or more additional sensors 106 included in the conductivity sensing device 104.

As further shown in FIG. 3, the probes 210 included in the conductivity sensor 204 extend from the bottom of the stainless-steel housing 300. In some examples, the probes 210 include an internal temperature sensor for compensation. In some examples, the stainless steel housing 300 can be fitted with a hidden gortex membrane vent 304 to equalize pressure and temperature within the housing 304, thereby reducing condensation effects on measurements generated by the conductivity sensing device 104.

As further shown in FIG. 3, the conductivity sensing device 104 can include a button 306 disposed on a top portion of the housing 300. The button 306 can be pressed to cycle the conductivity sensing device 104 through one or more operating modes such as, but not limited to, a wake mode, a sleep mode, and/or one or more other modes. The operating mode and/or operating status of the conductivity sensing device 104 can be displayed using a light emitting diode (LED) display 308 disposed on the button, the stainless steel housing, 300 and/or the removable top cover 302.

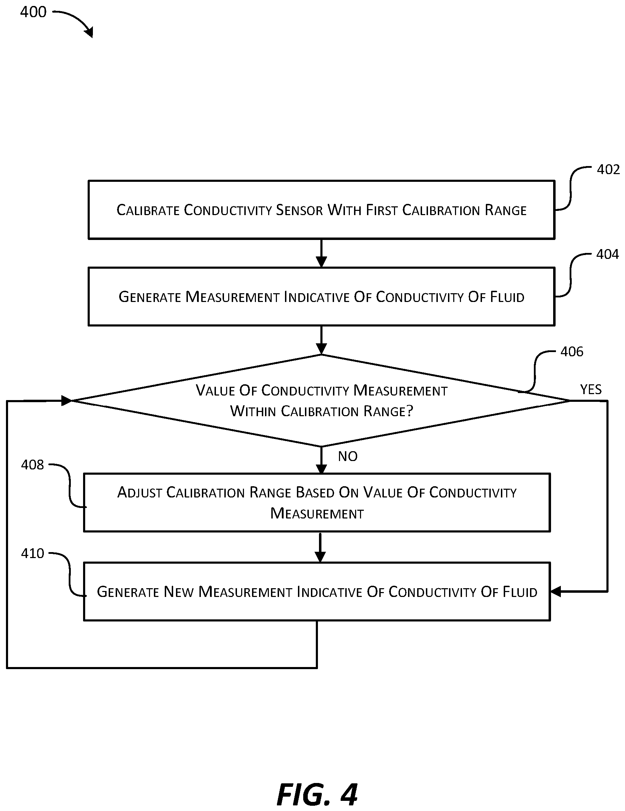

FIG. 4 is a flow diagram of method steps for monitoring the conductivity of fluid, according to various embodiments. Although the method steps are described in conjunction with FIGS. 1-3, persons skilled in the art will understand that any system configured to perform the method steps, in any order, is within the scope of the present disclosure.

As shown, a method 400 begins at step 402, at which a conductivity sensor is calibrated with a first of calibration range. For example, the processor 202 and/or the control unit 102 calibrates the conductivity sensing device 104 with a first calibration range.

At step 404, a first measurement indicative of the conductivity of a fluid is generated. For example, the conductivity sensing device 104 generates, using the conductivity sensor 204, a first measurement comprising a value indicative of the conductivity the fluid.

At step 406, a controller determines whether the value of the first measurement is within the first calibration range. For example, the processor 202 and/or the control unit 102 determines whether the value of the first measurement generated by the conductivity sensing device 104 is within the first calibration range.

If, at step 406, the controller determines that the value of the first measurement is within the first calibration range (YES), the method proceeds to step 410 where a new measurement indicative of the conductivity of the fluid is generated. For example, the conductivity sensing device 104 generates, via the conductivity sensor 204, a new measurement indicative of the conductivity of the fluid responsive to the processor 202 and/or the control unit 102 determining that the value of the first measurement is within the first calibration range.

However, if the controller determines that the value of the first measurement is not within the first calibration range (NO) at step 406, the method 400 proceeds to step 408 where the calibration range of the conductivity sensor is adjusted based on the value of the first measurement. For example, the conductivity sensing device 104 adjusts the calibration range of the conductivity sensing device 104 to include and/or center around the value of the first measurement responsive to the processor 202 and/or the control unit 102 determining that the value of the first measurement is not within the first calibration range. As described herein, the processor 202 and/or the control unit 102 can determine the new calibration points and/or range for the conductivity sensor 104 using the calibration data.

After adjusting the calibration range of the conductivity sensor, the method proceeds to step 410 where a new measurement indicative of the conductivity of the fluid is generated in accordance with the new calibration range. For example, the conductivity sensing device 104 generates a new measurement indicative of the conductivity of the fluid after the processor 202 and/or the control unit 102 adjusts the calibration range of the conductivity sensing device 104. After step 410, the method returns to step 406 where it is determined whether the value of the new conductivity measurement is within the current calibration range (e.g., either the first calibration range or the new adjusted calibration range) of the conductivity sensor.

In some examples, at step 406, the controller (e.g., the processor 202 or control unit 102) may instead determine whether the value of the conductivity measurement is (i) within a threshold amount of the center of the calibration range and/or (ii) whether the value of the conductivity measurement skews towards one of the bounds of the calibration range. In such examples, the controller can configure the conductivity sensing device 104 with a new calibration range 104 centered around the value of the conductivity measurement in response to determining that (i) the value of the conductivity measurement is not within a threshold amount of the center of the calibration range and/or (ii) the value of the conductivity measurement skews towards one of the bounds of the calibration range.

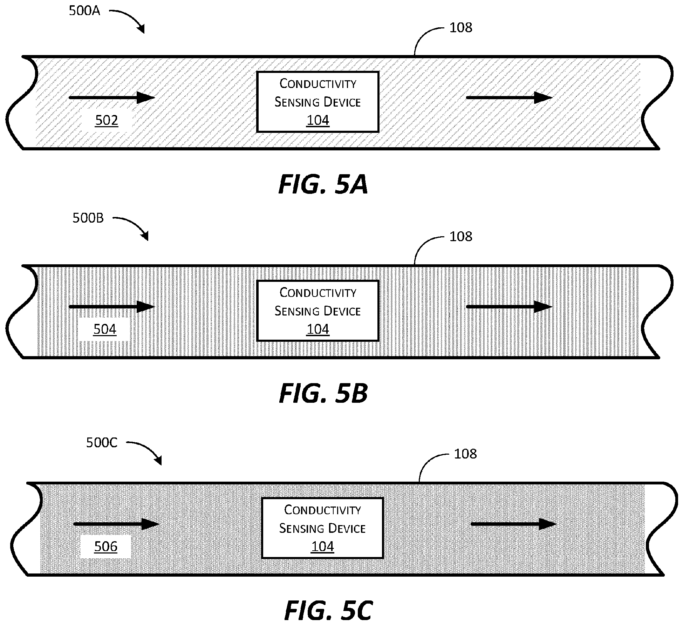

FIGS. 5A-5C illustrate an example process in which a conductivity sensor monitors multiple fluids flowing through a single pipeline, according to various embodiments. For example, FIGS. 5A-5C illustrates an example brewing process in which the conductivity sensing device 104 monitors the sequential flow of a first fluid (e.g., beer), a second fluid (e.g., rinse water), and a third fluid (e.g., cleaning solution) through the pipe 108. Although FIGS. 5A-5C are described herein with respect to a brewing process, persons skilled in the art should understand that the conductivity sensing device 104 can be used to monitor fluids flowing through the pipe 108 during one or more other types of processes.

As shown in FIG. 5A, a first fluid 502 flows through the pipe 108 during a first phase 500A of the brewing process. The first fluid 502 can be, for example, beer or some other type of beverage product. In some examples, prior to the first phase 500A of the brewing process, the processor 202 and/or the control unit 102 calibrates the conductivity sensing device with calibration points and/or a calibration range that corresponds to the first fluid 502. For example, if the first fluid 502 is beer, the processor 202 and/or the control unit 102 configures the conductivity sensing device 104 with moderate calibration points and/or a moderate calibration range that corresponds to the expected conductivity levels of beer.

In other examples, the processor 202 and/or the control unit 102 calibrates the conductivity sensing device 104 after the conductivity sensing device 104 generates a first conductivity measurement indicative of the conductivity of the first fluid 502. In such examples, the processor 202 and/or the control unit 102 determines, based on (i) the value of the first conductivity measurement and (ii) calibration data associated with the first fluid 502 (e.g., beer) that flows through the pipe 108 during the first phase 500A of the brewing process, a first set of calibration points and/or a first calibration range to configure the conductivity sensing device 104 with. Following calibration of the conductivity sensing device 104 with the first set of calibration points and/or the first calibration range, the conductivity sensing device 104 continues to generate conductivity measurements indicative of the conductivity of the first fluid during the first step 500A.

Eventually, the brewing process transitions from the first phase 500A to a second phase 500B in which a second fluid 504, such as rinse water, rinses the pipe 108 of the first fluid 502. FIG. 5B illustrates an example in which a second fluid 504 (e.g., rinse water) flows through the pipe 108 to remove the first fluid 502 (e.g., beer). As the second fluid 504 flows through the pipe 108, the conductivity sensing device 104 can generate a new conductivity measurement. However, as discussed above, the conductivity sensing device 104 is configured with a first set of calibration points and/or a first calibration range that corresponds to the expected conductivity of the first fluid 502 (e.g., beer), and thus, new conductivity measurements generated by the conductivity sensing device 104 while the second fluid 504 (e.g., rinse water) flows through the pipe 108 will likely comprise conductivity values that are outside of the first set of calibration points and/or the first calibration range.

For example, the conductivity sensing device 104 can generate a conductivity measurement having a conductivity value that is less than the first set of calibration points and/or the first calibration range associated with the first fluid 502 and/or the first phase 500A of the brewing process 500A. Responsive to determining that the value of a new conductivity measurement is less than the first calibration range and/or the first set of calibration points, the processor 202 and/or the control unit 102 can determine whether the decrease in the value of the conductivity measurement is attributed to a transition between phases (e.g., phases 500A and 500B) in the brewing process or a fault condition. For example, the processor 202 and/or the control unit 102 uses the decreased value of the conductivity measurement in combination with one or more of (i) calibration data indicative of the expected values for the conductivity of the second fluid 504, (ii) calibration data that provides additional context regarding the transition between phases 500A and 500B, and/or (iii) one or more measurements (e.g., temperature measurements, pressure measurements, vibration measurements, etc.) generated by the additional sensors 106 that may otherwise indicate a transition between phases or a fault condition.

If the processor 202 and/or the control unit 102 determines that the decrease in the value of the conductivity measurement is attributed to the transition between phases 500A and 500B of the brewing process, the processor 202 and/or the control unit 102 can configure the conductivity sensing device 104 with a second set of calibration points and/or a second calibration range that corresponds to the expected conductivity of the second fluid 504. For example, if the second fluid 504 is rinse water, the processor 202 and/or the control unit 102 configures the conductivity sensing device 104 with relatively low calibration points and/or a relatively low calibration range that corresponds to the expected conductivity levels of rinse water.

However, if the processor 202 and/or the control unit 102 determines that the decrease in the value of the conductivity measurement is not attributed to the transition between phases 500A and 500B of the brewing process, the processor 202 and/or the control unit 102 can generate an alert. As described herein, generating an alert can include one or more of activating a visual alarm, activating an audible alarm, and/or generating and transmitting an alert notification to the control unit 102 and/or some other remote computing device. The alert notification can be in the form of any suitable electronic message (e.g., email, text, short electronic messages (SMS), and/or others).

In some examples, the processor 202 and/or the control unit 102 determines that the decrease in the value of the conductivity measurement generated by the conductivity sensing device 104 is not attributed to a transition between phases 500A and 500B if context information included in the calibration data indicates that the brewing process should still be in the first phase 500A. In such examples, the decrease in the value of the conductivity measurement might instead be attributed to a water leak that is diluting the first fluid 500A. Accordingly, the processor 202 and/or the control unit 102 can generate an alert that indicates presence of a water leak.

Similar to the transition between phases 500A and 500B of the brewing process, the brewing process eventually transitions from the second phase 500B to a second phase 500C in which a third fluid 506, such as a cleaning solution, cleans the pipe 108. FIG. 5C illustrates an example in which a third fluid 506 (e.g., cleaning solution) flows through and cleans the pipe 108. As the third fluid 506 flows through the pipe 108, the conductivity sensing device 104 can generate a new conductivity measurement. However, as discussed above, the conductivity sensing device 104 might be configured with (i) a first set of calibration points and/or a first calibration range that corresponds to the expected conductivity of the first fluid 502 or (ii) a second set of calibration points and/or a second calibration range that corresponds to the expected conductivity of the second fluid 504. In that regard, conductivity measurements generated by the conductivity sensing device 104 while the third fluid 506 (e.g., cleaning solution) flows through the pipe 108 will likely comprise conductivity values that are outside of (i) the first set of calibration points and/or the first calibration range or (ii) the second set of calibration points and/or the second calibration range.

For example, the conductivity sensing device 104 can generate a conductivity measurement having a conductivity value that is greater than the set of calibration points and/or the calibration range with which the conductivity sensing device 104 is currently configured. Responsive to determining that the value of a new conductivity measurement is greater than the current calibration range and/or the current set of calibration points, the processor 202 and/or the control unit 102 can determine whether the increase in the value of the conductivity measurement is attributed to a transition between phases (e.g., phases 500B and 500C) in the brewing process or a fault condition. For example, to determine whether the increase in the value of the conductivity measurement is attributed to a transition between phases (e.g., phases 500B and 500C) in the brewing process or a fault condition, the processor 202 and/or the control unit 102 uses the increased value of the conductivity measurement in combination with one or more of (i) calibration data indicative of the expected values for the conductivity of the third fluid 506, (ii) calibration data that provides additional context regarding the transition between phases 500B and 500C, and/or (iii) one or more measurements (e.g., temperature measurements, pressure measurements, vibration measurements, etc.) generated by the additional sensors 106 that may otherwise indicate a transition between phases or a fault condition.

If the processor 202 and/or the control unit 102 determines that the increase in the value of the conductivity measurement is attributed to the transition between phases 500B and 500C of the brewing process, the processor 202 and/or the control unit 102 can configure the conductivity sensing device 104 with a third set of calibration points and/or a third calibration range that corresponds to the expected conductivity of the third fluid 506. For example, if the third fluid 506 is a cleaning solution, the processor 202 and/or the control unit 102 configures the conductivity sensing device 104 with relatively high calibration points and/or a relatively high calibration range that corresponds to the expected conductivity levels of the cleaning solution.

However, if the processor 202 and/or the control unit 102 determines that the increase in the value of the conductivity measurement is not attributed to the transition between phases 500B and 500C of the brewing process, the processor 202 and/or the control unit 102 can generate an alert. As described herein, generating an alert can include one or more of activating a visual alarm, activating an audible alarm, and/or generating and transmitting an alert notification to the control unit 102 and/or some other remote computing device. The alert notification can be in the form of any suitable electronic message (e.g., email, text, short electronic messages (SMS), and/or others).

In some examples, the processor 202 and/or the control unit 102 determines that the increase in the value of the conductivity measurement generated by the conductivity sensing device 104 is not attributed to a transition between phases 500B and 500C if context information included in the calibration data indicates that the brewing process should still be in the first phase 500A or the second phase 500B. In such examples, the increase in the value of the conductivity measurement might instead be attributed to contamination of the first fluid 502 or the second fluid 504 with a fluid that is highly conductive. Accordingly, the processor 202 and/or the control unit 102 can generate an alert that indicates the presence of fluid contamination. In some examples, the fluid contamination is caused by a heat exchanger leak.

In brewery operations and other beverage manufacturing environments, heat exchangers are widely used to control the temperature of process fluids such as wort, beer, or cleaning solutions. Heat exchangers typically operate by transferring heat between two separate fluid circuits - for example, between chilled glycol and product streams - without allowing the fluid circuits to physically mix. However, over time, degradation of the heat exchanger plates or gaskets can lead to cross-contamination through micro-leaks or ruptures, potentially compromising product quality and safety.

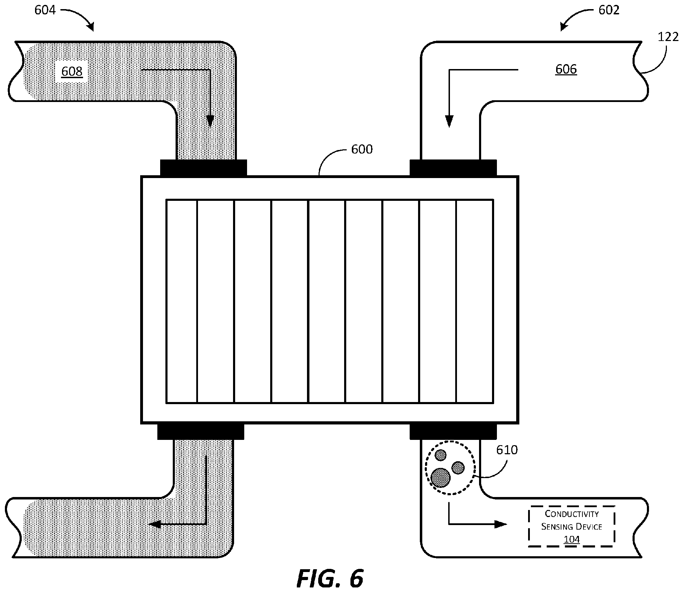

FIG. 6 illustrates an example in which a conductivity sensor detects a leak from a heat exchanger, according to various embodiments. As shown in FIG. 6, a heat exchanger 600 is adapted to transfer heat between a first fluid circuit 602 and a second fluid circuit 604. The first fluid circuit 602 transports a product fluid 606 (e.g., beer or some other beverage product) through the heat exchanger 600 and the second fluid circuit 604 transports a refrigerant fluid 608 (e.g., chilled glycol or some other type of refrigerant) through the heat exchanger 600 such that the product fluid 606 is chilled by the refrigerant fluid 608 without mixing.

However, as described above, degradation of the heat exchanger 600 over time can lead to cross-contamination between the product fluid 606 and the refrigerant fluid 608 through micro-leak ruptures in one or more components of the heat exchanger 600. In the illustrated example of FIG. 6, the product fluid 606 is contaminated with refrigerant fluid sample 610 that has leaked into the product fluid 606. With the techniques described herein, the processor 202 and/or the control unit 102 can detect the presence of the refrigerant fluid sample 610 within the product fluid 606 to help mitigate further damage to the quality and/or safety of the product fluid 606. In some examples, the processor 202 and/or control unit 102 can detect whether refrigerant fluid sample 610 is present within the product fluid 606 based on (i) a value of a conductivity measurement generated by the conductivity sensing device 104, (ii) calibration data that indicates the expected conductivity values of the product fluid 606 and refrigerant fluid 608, and/or (iii) other contextual information associated with the process involving the product fluid 606. As an example, the processor 202 and/or the control unit 102 can determine that the heat exchanger 600 has leaked refrigerant fluid 608 into the product fluid 606 in response to determining one or more of (i) that the value of the conductivity measurement exceeds a current calibration range and/or current calibration points of the conductivity sensing device 104 and/or (ii) that the value of the conductivity measurement exceeds expected values for the conductivity of the product fluid 606 for the current phase of the process involving the product fluid 606. In response to determining that the heat exchanger 600 has leaked refrigerant fluid 608 into the product fluid 606, the processor 202 and/or the control unit 102 can generate and/or transmit an alert that indicates the occurrence of a heat exchanger leak.

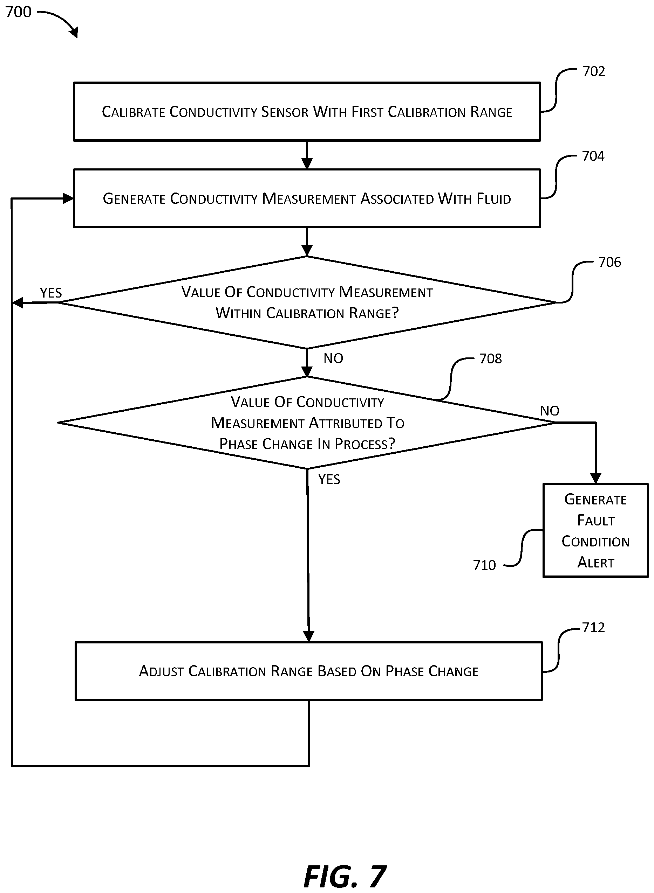

FIG. 7 is a flow diagram of method steps for monitoring the conductivity of fluid during a fluid manufacturing process, according to various embodiments. Although the method steps are described in conjunction with FIGS. 1-6, persons skilled in the art will understand that any system configured to perform the method steps, in any order, is within the scope of the present disclosure.

As shown, a method 700 begins at step 702, at which a conductivity sensor is calibrated with a first of calibration range. For example, the processor 202 and/or the control unit 102 calibrates the conductivity sensing device 104 with a first calibration range.

At step 704, a conductivity measurement associated with a fluid is generated. For example, the conductivity sensing device 104 generates, using the conductivity sensor 204, measurement indicative of the conductivity of a fluid.

At step 706, a controller determines whether the value of the conductivity measurement is within the first calibration range. For example, the processor 202 and/or the control unit 102 determines whether the value of the conductivity measurement generated by the conductivity sensing device 104 is within the first calibration range.

If, at step 706, the controller determines that the value of the conductivity measurement is within the first calibration range (YES), the method returns to step 704 where a new measurement indicative of the conductivity of the fluid is generated. For example, the conductivity sensing device 104 generates, via the conductivity sensor 204, a new measurement indicative of the conductivity of the fluid responsive to the processor 202 and/or the control unit 102 determining that the value of the first measurement is within the first calibration range.

However, if the controller determines that the value of the first measurement is not within the first calibration range (NO) at step 706, the method 700 proceeds to step 708 where the controller determines whether the value of the conductivity measurement is attributed to phase change in the process involving the fluid. For example, the processor 202 and/or the control unit 102 can determine whether a transition between phases in the process involving the fluid has caused the value of the conductivity measurement to fall outside of the first calibration range. As described herein, the processor 202 and/or the control unit 102 can use one or more of the value of the conductivity measurement, calibration data indicative of the expected values for the conductivity of fluids during a first phase in the process, calibration data indicative of the expected values for the conductivity of fluids during a second phase in the process, one or more measurements generated by one or more additional sensors 106 that may otherwise indicate a transition between phases or a fault condition, and/or any other type of contextual information regarding the current phase in the process to determine whether a transition between phases in the process involving the fluid has caused the value of the conductivity measurement to fall outside of the first calibration range

If, at step 708, the controller determines that the value of the conductivity measurement is not attributed to a phase change in the process (NO), the method proceeds to step 710 where an alert indicative of a fault condition is generated. For example, the processor 202 and/or the control unit 102 generates an alert that indicates a fault condition has caused the conductivity of the fluid to deviate from expected values. The fault condition can be, for example, a leak of refrigerant fluid from a heat exchanger into the fluid.

If, at step 708, the controller determines that the value of the conductivity measurement is attributed to a phase change in the process (YES), the method proceeds to step 712 where the calibration range of the conductivity sensor is adjusted based on the phase change. For example, the processor 202 and/or the control unit 102 adjusts the calibration range of the conductivity sensing device 104 to correspond to expected conductivity values of a fluid during the phase that the process has just transitioned to. For example, if the processor 202 and/or the control unit 102 determines that a transition to a rinse phase has caused the value of the conductivity measurement to deviate from the current calibration range of the conductivity sensing device 104, the processor 202 and/or the control unit 102 adjusts the calibration range of the conductivity sensing device 104 to correspond to the expected conductivity values of fluid during the rinse phase at step 712. After step 712, the method returns to step 704 where a new measurement indicative of the conductivity of the fluid is generated.

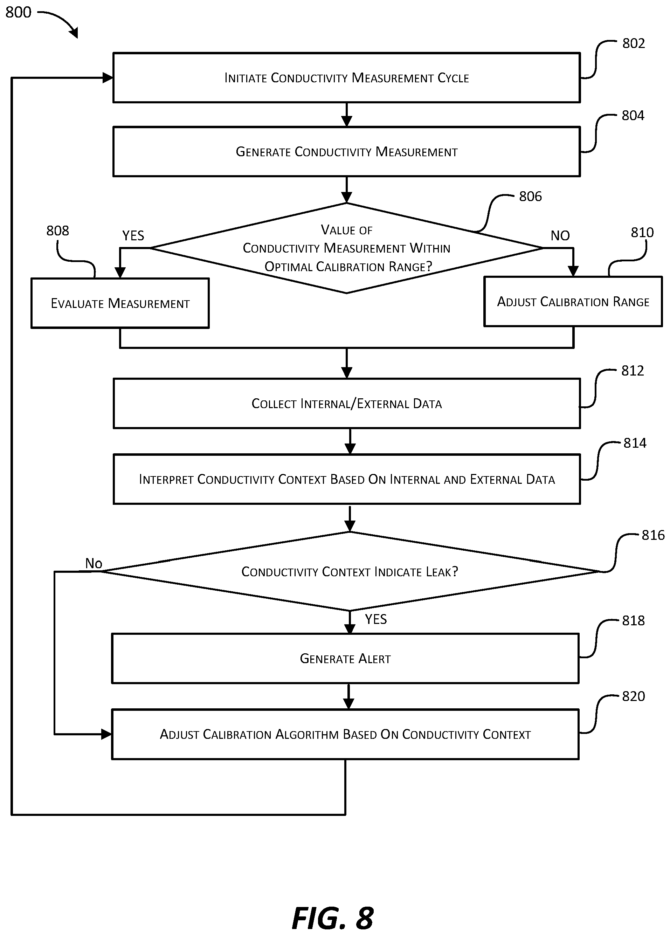

FIG. 8 is a flow diagram of method steps for calibrating a conductivity sensor, according to various embodiments. Although the method steps are described in conjunction with FIGS. 1-7, persons skilled in the art will understand that any system configured to perform the method steps, in any order, is within the scope of the present disclosure.

As shown, a method 800 begins at step 802, at which a conductivity measurement cycle is initiated. At step 804, a conductivity measurement associated with a fluid is generated. For example, the conductivity sensing device 104 generates, using the conductivity sensor 204, a measurement indicative of the conductivity of a fluid.

At step 806, a controller determines whether the value of the conductivity measurement is within an optimal calibration range for conductivity sensing device 104. For example, the processor 202 and/or the control unit 102 determines whether the value of the conductivity measurement generated by the conductivity sensing device 104 is within an optimal calibration range.

If, at step 806, the controller determines that the value of the conductivity measurement is within the first calibration range (YES), the method 800 proceeds to step 808 where the conductivity measurement is evaluated. However, if the controller determines that the value of the first measurement is not within the first calibration range (NO) at step 806, the method 800 proceeds to step 810where the controller adjusts the calibration range of the conductivity sensor. For example, the processor 202 and/or the control unit 102 adjusts the calibration range of the conductivity sensing device 104 using one or more techniques described herein.

At step 812, data internal and/or external to the conductivity sensor is collected. For example, the processor 202 and/or the control unit 102 collects data internal to the conductivity sensing device 104 (e.g., vibration data) and data external to the conductivity sensing device 104 (e.g., temperature data, pressure data, pump data, etc.) from one or more additional sensors 106 and/or other remote computing devices.

At step 814, the context of the conductivity measurement is interpreted based in part on the data internal and/or external to the conductivity sensor. For example, the processor 202 and/or the control unit 102 determines a context surrounding the value of the conductivity measurement based in part on data internal to the conductivity sensing device 104 (e.g., vibration data) and data external to the conductivity sensing device 104 (e.g., temperature data, pressure data, pump data, etc.). The context surrounding the value of the conductivity measurement can indicate one or more of a current phase in the process, a transition between phases in the process, a drift in conductivity values over time, a fault condition, and/or some other type of information associated with the value of the conductivity measurement.

At step 816, a controller determines whether the context of the conductivity measurement indicates the presence of a leak (e.g., a refrigerant leak, a water leak, etc.). For example, the processor 202 and/or the control unit 102 determines whether a leak is present based on the context of the conductivity measurement generated by the conductivity sensing device 104.

If, at step 816, the controller determines that a leak is present (YES), the method 800 proceeds to step 818 where an alert is generated. However, if the controller determines that a leak is not present (NO) at step 816, the method 800 proceeds to step 820. At step 820, the controller (e.g., the processor 202 and/or the control unit 102) adjusts a calibration algorithm based on the context of the conductivity measurement. Then, following step 820, the method 800 returns to step 802 where a new measurement cycle is initiated.

Although certain aspects have been described with reference to certain examples, variations and modifications exist within the spirit and scope of one or more independent aspects. Various features and aspects are set forth in the following claims.

Any and all combinations of any of the claim elements recited in any of the claims and/or any elements described in this application, in any fashion, fall within the contemplated scope of the present disclosure and protection. The descriptions of the various embodiments have been presented for purposes of illustration, but are not intended to be exhaustive or limited to the embodiments disclosed. Many modifications and variations will be apparent to those of ordinary skill in the art without departing from the scope and spirit of the described embodiments.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

Aspects of the present disclosure are described above with reference to flowchart illustrations and/or block diagrams of methods, apparatus (systems) and computer program products according to embodiments of the disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine.

The instructions, when executed via the processor of the computer or other programmable data processing apparatus, enable the implementation of the functions/acts specified in the flowchart and/or block diagram block or blocks. Such processors may be, without limitation, general purpose processors, special-purpose processors, application-specific processors, or field-programmable gate arrays.

The flowchart and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

While the preceding is directed to embodiments of the present disclosure, other and further embodiments of the disclosure may be devised without departing from the basic scope thereof, and the scope thereof is determined by the claims that follow.

## Claims

1. A method for monitoring conductivity of a fluid, the method comprising:
calibrating a conductivity sensor with a first calibration range;
generating a first measurement indicative of the conductivity of the fluid;
determining whether a value of the first measurement is within the first calibration range;
responsive to determining that the value of the first measurement is not within the first calibration range, calibrating the conductivity sensor with a second calibration range that includes the value of the first measurement; and
generating a second measurement indicative of the conductivity of the fluid.

2. The method of claim 1, further comprising:
generating a third measurement indicative of the conductivity of the fluid;
determining whether a value of the third measurement is within the second calibration range;
responsive to determining that the value of the third measurement is not within the second calibration range, determining whether the value of the third measurement is attributed to a transition from a first phase in a process involving the fluid to a second phase in the process involving the fluid; and
responsive to determining that the value of the third measurement is attributed to the transition, calibrating the conductivity sensor with a third calibration range that corresponds to the second phase in the process.

3. The method of claim 2, wherein the first fluid is beer and the second fluid is rinse water.

4. The method of claim 1, wherein determining whether the value of the third measurement is attributed to the transition includes:
receiving vibration data and temperature data from one or more additional sensors; and
determining, based in part on the vibration data and the temperature data, that the value of the third measurement is attributed to the transition.

5. The method of claim 1, further comprising:
generating a third measurement indicative of the conductivity of the fluid;
determining whether a value of the third measurement is within the second calibration range;
responsive to determining that the value of the third measurement is not within the second calibration range, determining whether the value of the third measurement is attributed to a leak; and
responsive to determining that the value of the third measurement is attributed to the leak, generating an alert.

6. The method of claim 5, wherein determining whether the value of the third measurement is attributed to the leak includes:
determining whether the value of the third measurement is greater than an expected conductivity value by a threshold amount; and
responsive to determining that the value of the third measurement is greater than the expected conductivity value by the threshold amount, determining that refrigerant from a heat exchanger has leaked into the fluid.

7. The method of claim 6, wherein the alert includes an indication that a heat exchanger has leaked.

8. The method of claim 5, wherein determining whether the value of the third measurement is attributed to the leak includes:
determining whether the value of the third measurement is less than an expected conductivity value by a threshold amount; and
responsive to determining that the value of the third measurement is less than the expected conductivity value by the threshold amount, determining that the fluid has been diluted by a water leak.

9. The method of claim 8, wherein the alert indicates the water leak.

10. The method of claim 1, wherein calibrating the conductivity sensor with the first calibration range includes received calibration points from a remote computing device.

11. A conductivity monitoring system, comprising:
a control unit; and
a conductivity sensing device in electronic communication with the control unit, the conductivity sensing device including a conductivity sensor and a processor adapted to:
receive calibration data from the control unit;
calibrate, based on the calibration data, the conductivity sensor with a first calibration range;
receive a first measurement indicative of the conductivity of a fluid from the conductivity sensor;
determine whether a value of the first measurement is within the first calibration range; and
responsive to determining that the value of the first measurement is not within the first calibration range, calibrating a conductivity sensor with a second calibration range that includes the value of the first measurement.

12. The system of claim 11, wherein the processor is further adapted to:
receive a second measurement indicative of the conductivity of the fluid from the conductivity sensor;
determine whether a value of the second measurement is within the second calibration range;
responsive to determining that the value of the second measurement is not within the second calibration range, determine whether the value of the second measurement is attributed to a transition from a first phase in a process involving the fluid to a second phase in the process involving the fluid; and
responsive to determining that the value of the second measurement is attributed to the transition, calibrate the conductivity sensor with a third calibration range that corresponds to the second phase in the process.

13. The system of claim 12, wherein the first fluid is rinse water and the second fluid is cleaning solution.

14. The system of claim 11, further comprising a vibration sensor and a temperature sensor; and
wherein to determine whether the value of the second measurement is attributed to the transition, the processor is further adapted to:
receive vibration data from the vibration sensor;
receive temperature data from the temperature sensor; and
determine, based in part on the vibration data and the temperature data, that the value of the second measurement is attributed to the transition.

15. The system of claim 11, wherein the processor is further adapted to:
receive a second measurement indicative of the conductivity of the fluid from the conductivity sensor;
determine whether a value of the second measurement is within the second calibration range;
responsive to determining that the value of the second measurement is not within the second calibration range, determine whether the value of the second measurement is attributed to a leak; and
responsive to determining that the value of the second measurement is attributed to the leak, generate an alert.

16. The system of claim 15, wherein to determine whether the value of the second measurement is attributed to the leak, the processor is further adapted to:
determine whether the value of the second measurement is greater than an expected conductivity value by a threshold amount; and
responsive to determining that the value of the second measurement is greater than the expected conductivity value by the threshold amount, determine that refrigerant from a heat exchanger has leaked into the fluid.

17. The system of claim 15, wherein to determine whether the value of the third measurement is attributed to the leak includes, the processor is further adapted to:
determine whether the value of the second measurement is less than an expected conductivity value by a threshold amount; and
responsive to determining that the value of the second measurement is less than the expected conductivity value by the threshold amount, determine that the fluid has been diluted by a water leak.

18. A conductivity sensing device, comprising:
a conductivity sensor adapted to sense a conductivity value of a fluid;
a memory device adapted to store calibration data; and
a processor in electronic communication with the conductivity sensor and the memory device, the processor adapted to:
calibrate, based on the calibration data, the conductivity sensor with a first calibration range;
receive the conductivity value of the fluid from the conductivity sensor;
determine whether the conductivity value of the fluid is within the first calibration range; and
responsive to determining that the conductivity value of the fluid is not within the first calibration range, calibrate a conductivity sensor with a second calibration range that includes the conductivity value of the fluid.

19. The conductivity sensing device of claim 18, further comprising a stainless steel housing adapted to protect the processor and the memory device.

20. The conductivity sensing device of claim 18, further comprising a vibration sensor and a temperature sensor.
